# EUROPÄISCHE PATENTANMELDUNG

(11) **EP 4 599 860 A1**
(43) Veröffentlichungstag der Anmeldung: **13.08.2025**
(21) Anmeldenummer: 25155470.5
(22) Anmeldetag: 03.02.2025
(51) Int. Cl.: A61M 1/14, A61M 1/16, A61M 1/36, G16H 40/63

(54) **VERFAHREN, DIALYSEMASCHINE UND DIALYSESYSTEM**

(30) Priorität: 09.02.2024 DE 102024103671
(71) Anmelder: B. Braun Avitum AG, 34212 Melsungen (DE)
(72) Erfinder: Janik, Waldemar, 34212 Melsungen (DE); Krause, Silvie, 34212 Melsungen (DE); Schellhase, Meik, 36179 Bebra (DE)
(74) Vertreter: Patentanwälte Ruff, Wilhelm, Beier, Dauster & Partner mbB

(57) **Zusammenfassung**

Die Erfindung betrifft ein Verfahren zum Betreiben einer Dialysemaschine (1), wobei die Dialysemaschine (1) aufweist: einen Normal-Betriebsmodus (NM), in welchem die Dialysemaschine (1) einen Normal-Bedarf (NB) an wenigstens einer der Dialysemaschine (1) zugeführten Betriebsressource (R) benötigt, und einen Eco-Betriebsmodus (EM), in welchem die Dialysemaschine (1) einen relativ zu dem Normal-Bedarf (NB) reduzierten Eco-Bedarf (EB) an der wenigstens einen der Dialysemaschine (1) zugeführten Betriebsressource (R) benötigt; wobei das Verfahren den Schritt aufweist: Betreiben der Dialysemaschine (1) in Abhängigkeit von wenigstens einem Kriterium (K) entweder in dem Normal-Betriebsmodus (NM) oder in dem Eco-Betriebsmodus (EM).

## Beschreibung

Die Erfindung betrifft ein Verfahren zum Betreiben einer Dialysemaschine sowie eine derartige Dialysemaschine. Zudem betrifft die Erfindung ein eine solche Dialysemaschine aufweisendes Dialysesystem.

Einer gewöhnlichen Dialysemaschine wird zu ihrem Betrieb typischerweise wenigstens eine Betriebsressource zugeführt. Eine Zufuhrmenge der Betriebsressource wird dabei im Betrieb der Dialysemaschine typischerweise nicht bedarfsgerecht angepasst.

Es ist eine Aufgabe der vorliegenden Erfindung, ein Verfahren zum Betreiben einer Dialysemaschine sowie eine derartige Dialysemaschine und ein eine solche Dialysemaschine aufweisendes Dialysesystem zu schaffen, die, insbesondere jeweils, verbesserte Eigenschaften aufweisen. Insbesondere soll ein besonders ressourceneffizienter Betrieb der Dialysemaschine ermöglicht werden.

Diese Aufgabe wird durch die Gegenstände der unabhängigen Patentansprüche gelöst. Bevorzugte Ausführungsformen sind Gegenstand der abhängigen Patentansprüche.

Ein erfindungsgemäßes Verfahren dient zum Betreiben einer Dialysemaschine. Die Dialysemaschine weist einen Normal-Betriebsmodus auf. In dem Normal-Betriebsmodus benötigt die Dialysemaschine einen Normal-Bedarf an wenigstens einer der Dialysemaschine zugeführten Betriebsressource. Zweckmäßig wird der benötigte Normal-Bedarf in dem Normal-Betriebsmodus verbraucht. Die Dialysemaschine weist einen Eco-Betriebsmodus auf. In dem Eco-Betriebsmodus benötigt die Dialysemaschine einen Eco-Bedarf an der wenigstens einen der Dialysemaschine zugeführten Betriebsressource. Zweckmäßig wird der Eco-Bedarf in dem Eco-Betriebsmodus verbraucht. Der Eco-Bedarf ist relativ zu dem Normal-Bedarf reduziert. Der Eco-Bedarf ist also insbesondere kleiner als der Normal-Bedarf. Das Verfahren weist einen Schritt auf, gemäß welchem ein Betreiben der Dialysemaschine in Abhängigkeit von wenigstens einem Kriterium entweder in dem Normal-Betriebsmodus oder in dem Eco-Betriebsmodus erfolgt. Auf diese Weise kann die Dialysemaschine in Abhängigkeit von dem wenigstens einen Kriterium, insbesondere bedarfsgerecht, besonders ressourceneffizient betrieben werden.

Zweckmäßig handelt es sich bei der wenigstens einen Betriebsressource um elektrische Energie sowie - alternativ oder zusätzlich - um eine Betriebsflüssigkeit, insbesondere aufweisend oder bestehend aus Wasser.

Zweckmäßig kann das Verfahren wenigstens teilweise, insbesondere schrittweise, computerimplementiert sein.

Zweckmäßig kann der Eco-Betriebsmodus synonym als "Ressourcenspar-Betriebsmodus" verstanden werden, insbesondere weil er eine Einsparung der wenigstens einen Betriebsressource relativ zu dem Normal-Betriebsmodus ermöglicht. Mit anderen Worten: Der Eco-Betriebsmodus kann, insbesondere bedarfsgerecht, einen im Vergleich zu dem Normal-Betriebsmodus besonders ressourceneffizienten Betrieb der Dialysemaschine ermöglichen.

In Ausgestaltung der Erfindung weist das Verfahren einen Schritt auf, gemäß welchem ein Überwachen des wenigstens einen Kriteriums für ein Aktivieren entweder des Normal-Betriebsmodus oder des Eco-Betriebsmodus erfolgt. Das Verfahren weist zudem einen Schritt auf, gemäß welchem ein Aktivieren entweder des Normal-Betriebsmodus oder des Eco-Betriebsmodus in Abhängigkeit von dem wenigstens einen Kriterium erfolgt, um die Dialysemaschine anschließend in Abhängigkeit von dem wenigstens einen Kriterium entweder in dem Normal-Betriebsmodus oder in dem Eco-Betriebsmodus zu betreiben. Insbesondere kann also die Dialysemaschine direkt mit Beginn ihres Betriebs in Abhängigkeit von dem wenigstens einen Kriterium entweder in ihrem Normal-Betriebsmodus oder in ihrem Eco-Betriebsmodus betrieben werden. Zweckmäßig kann das Aktivieren entweder des Normal-Betriebsmodus oder des Eco-Betriebsmodus manuell sowie - alternativ oder zusätzlich - automatisch erfolgen. Es ist also denkbar, dass das Aktivieren entweder nur automatisch oder nur manuell oder sowohl automatisch als auch manuell möglich ist.

Zweckmäßig können mehrere Kriterien überwacht werden. Falls mehrere Kriterien überwacht werden, können einige oder alle dieser Kriterien unterschiedlich stark oder gleich stark gewichtet werden.

In weiterer Ausgestaltung der Erfindung weist das Verfahren einen Schritt auf, gemäß welchem ein Überwachen des wenigstens einen Kriteriums für ein Umschalten zwischen dem Normal-Betriebsmodus und dem Eco-Betriebsmodus erfolgt. Zudem weist das Verfahren einen Schritt auf, gemäß welchem ein Umschalten zwischen den Normal-Betriebsmodus und dem Eco-Betriebsmodus in Abhängigkeit von dem wenigstens einen überwachten Kriterium erfolgt, so dass die Dialysemaschine anschließend in Abhängigkeit von dem wenigstens einen Kriterium entweder in dem Normal-Betriebsmodus oder in dem Eco-Betriebsmodus betrieben wird. Auf diese Weise kann während des Betriebs der Dialysemaschine der Bedarf an der wenigstens einen Betriebsressource angepasst werden, was Vorteile hinsichtlich der Ressourceneffizienz bedingen kann. Zweckmäßig resultiert aus dem Umschalten ein Wechsel zwischen den Betriebsmodi.

In weiterer Ausgestaltung der Erfindung erfolgt das Umschalten zwischen dem Normal-Betriebsmodus und dem Eco-Betriebsmodus automatisch. Alternativ oder zusätzlich erfolgt das Umschalten zwischen dem Normal-Betriebsmodus und dem Eco-Betriebsmodus manuell. Es ist also denkbar, dass das Umschalten entweder nur automatisch oder nur manuell oder sowohl automatisch als auch manuell möglich ist.

In weiterer Ausgestaltung der Erfindung wird bei dem Betreiben der Dialysemaschine, insbesondere sowohl dem Normal-Betriebsmodus als auch in dem Eco-Betriebsmodus, ein Dialysat mit einer Dialysatflussrate durch die Dialysemaschine geführt. Die Dialysatflussrate kann, insbesondere sowohl dem Normal-Betriebsmodus als auch in dem Eco-Betriebsmodus, 100 ml/min bis 800 ml/min betragen. Dabei ist die Dialysatflussrate, insbesondere beidenfalls innerhalb der vorgenannten Range, in dem Eco-Betriebsmodus kleiner als in dem Normal-Betriebsmodus.

Zweckmäßig entspricht die Dialysatflussrate in dem Eco-Betriebsmodus einem 1,0-Fachen bis 1,6-Fachen einer Blutflussrate von Blut, welches mittels der Dialysemaschine dialysiert wird und/oder dialysierbar ist. Insbesondere entspricht in dem Eco-Betriebsmodus die Dialysatflussrate einem 1,0-Fachen bis 1,2-Fachen der Blutflussrate. Auf diese Weise können Ergebnisse von Studien ausgenutzt werden, welche gezeigt haben, dass eine Erhöhung des Dialysatflusses keine wesentlich höhere Dialyseeffektivität zur Folge hat und umgekehrt eine Reduzierung des Dialysatflusses die Dialyseeffektivität nicht wesentlich verringert, insbesondere mit Bezug auf eine häufig eingestellte Dialysatflussrate von etwa 500 ml/min.

In weiterer Ausgestaltung der Erfindung weist die Dialysemaschine eine, insbesondere berührungssensitive, elektronische Anzeigeeinrichtung zur Visualisierung eines momentanen Betriebsmodus der Dialysemaschine auf. Der momentane Betriebsmodus kann insbesondere der momentan aktivierte Betriebsmodus der Dialysemaschine sein. In dem Eco-Betriebsmodus ist eine Anzeigehelligkeit der Anzeigeeinrichtung relativ zu dem Normal-Betriebsmodus geringer. Die Anzeigehelligkeit kann also in dem Eco-Betriebsmodus relativ zu dem Normal-Betriebsmodus gedimmt sein. Alternativ oder zusätzlich ist ein Farbschema der Anzeigeeinrichtung in dem Eco-Betriebsmodus relativ zu dem Normal-Betriebsmodus angepasst. Insbesondere wenn die Anzeigeeinrichtung ein OLED-Display aufweist, kann die Anzeigeeinrichtung in dem Normal-Betriebsmodus ein buntes Farbschema aufweisen, wohingegen die Anzeigeeinrichtung in dem Eco-Betriebsmodus ein unbuntes, insbesondere schwarz-weißes, Farbschema aufweist. Insbesondere da bei OLED-Displays die Farbe Schwarz durch unbeleuchtete und somit ausgeschaltete Pixel dargestellt werden kann, kann auf diese Weise elektrische Energie eingespart werden. Alternativ oder zusätzlich ist die Anzeigeeinrichtung in dem Eco-Betriebsmodus relativ zu dem Normal-Betriebsmodus, insbesondere vollständig, deaktiviert. Insbesondere wird mittels der deaktivierten Anzeigeeinrichtung der momentane Betriebsmodus zeitweise nicht visualisiert.

In weiterer Ausgestaltung der Erfindung weist die Dialysemaschine eine Sensoreinrichtung auf. Insbesondere ist die Sensoreinrichtung zum Detektieren einer Größe für das wenigstens eine Kriterium ausgebildet. Die Größe für das wenigstens eine Kriterium kann entweder eine Erfüllung des wenigstens einen Kriteriums direkt wiedergeben oder die Erfüllung des wenigstens einen Kriteriums kann infolge eines Vergleichs der Größe mit einem zugehörigen Schwellwert erkannt werden. Die Sensoreinrichtung weist einen Helligkeitssensor auf, der zum Detektieren einer Umgebungshelligkeit der Dialysemaschine ausgebildet ist. Die Umgebungshelligkeit kann eine Größe für das wenigstens eine Kriterium sein. Alternativ oder zusätzlich kann das wenigstens eine Kriterium ein vorbestimmter Schwellwert der Umgebungshelligkeit sein. Die Sensoreinrichtung weist alternativ oder zusätzlich einen Näherungssensor auf, der zum Detektieren einer sich der Dialysemaschine nähernden Person ausgebildet ist. Dabei kann ein Abstand der Person zu der Dialysemaschine eine Größe für das wenigstens eine Kriterium sein. Alternativ oder zusätzlich kann das wenigstens eine Kriterium ein vorbestimmter Schwellwert des Abstands sein. Die Sensoreinrichtung weist alternativ oder zusätzlich einen Berührungssensor auf, der zum Detektieren einer berührenden, insbesondere manuellen, Eingabe einer Größe für das wenigstens eine Kriterium ausgebildet ist. Der Berührungssensor kann von der berührungssensitiven Anzeigeeinrichtung, insbesondere integral und/oder funktional, umfasst sein.

In weiterer Ausgestaltung der Erfindung weist die Dialysemaschine einen in dem Normal-Betriebsmodus und in dem Eco-Betriebsmodus, insbesondere je wahlweise und/oder zeitweise, aktivierbaren Freispülmodus auf. Der Freispülmodus dient zum Freispülen der Dialysemaschine mit einer Freispülflüssigkeit. Die Freispülflüssigkeit kann die Betriebsflüssigkeit aufweisen oder sein. Wenn der Freispülmodus bei dem Betreiben der Dialysemaschine in dem Normal-Betriebsmodus aktiviert wird, wird die Freispülflüssigkeit, insbesondere bei Wärmezufuhr mittels einer elektrischen Heizeinrichtung der Dialysemaschine, mit einer Normal-Freispültemperatur und mit einer Freispülflüssigkeitsflussrate durch die Dialysemaschine geführt. Die Normal-Freispültemperatur kann 40 °C betragen. Die Freispülflüssigkeitsflussrate kann 1 l/min betragen. Wenn der Freispülmodus bei dem Betreiben der Dialysemaschine in dem Eco-Betriebsmodus aktiviert wird, wird die Freispülflüssigkeit, insbesondere ohne Wärmezufuhr mittels der elektrischen Heizeinrichtung, mit einer gegenüber der Normal-Freispültemperatur reduzierten Eco-Freispültemperatur und mit der, insbesondere gleichen, Freispülflüssigkeitsflussrate durch die Dialysemaschine geführt. Die Eco-Freispültemperatur kann einer Temperatur der Freispülflüssigkeit entsprechen, die die Freispülflüssigkeit bei Entnahme aus einem Freispülflüssigkeits-Zufuhrsystem für die Dialysemaschine aufweist. Beispielsweise kann die Eco-Freispültemperatur 15 °C betragen. Die Reduzierung der Freispültemperatur kann eine Energieeinsparung ermöglichen. Zweckmäßig erfolgt ein Aktivieren und/oder ein Umschalten des Freispülmodus in Abhängigkeit von dem wenigstens einen Kriterium und/oder zeitgesteuert.

In weiterer Ausgestaltung der Erfindung weist die Dialysemaschine einen in dem Normal-Betriebsmodus und in dem Eco-Betriebsmodus, insbesondere je wahlweise und/oder zeitweise, aktivierbaren Heißreinigungsmodus auf, wobei der Heißreinigungsmodus zum Heißreinigen der Dialysemaschine mit einer Heißreinigungsflüssigkeit dient. Die Heißreinigungsflüssigkeit kann die Betriebsflüssigkeit aufweisen oder sein. Wenn der Heißreinigungsmodus bei dem Betreiben der Dialysemaschine in dem Normal-Betriebsmodus aktiviert wird, wird die Heißreinigungsflüssigkeit, insbesondere bei Wärmezufuhr mittels der elektrischen Heizeinrichtung der Dialysemaschine, mit einer Normal-Heißreinigungstemperatur und mit einer Normal-Heißreinigungsdauer durch die Dialysemaschine geführt. Wenn der Heißreinigungsmodus bei dem Betreiben der Dialysemaschine in dem Eco-Betriebsmodus aktiviert wird, wird die Heißreinigungsflüssigkeit, insbesondere bei Wärmezufuhr mittels der elektrischen Heizeinrichtung, mit einer gegenüber der Normal-Heißreinigungstemperatur reduzierten Eco-Heißreinigungstemperatur und mit einer gegenüber der Normal-Heißreinigungsdauer verlängerten Eco-Heißreinigungsdauer durch die Dialysemaschine geführt. Das Reduzieren der Heißreinigungstemperatur kann eine Energieeinsparung ermöglichen. Zweckmäßig erfolgt ein Aktivieren und/oder ein Umschalten des Heißreinigungsmodus in Abhängigkeit von dem wenigstens einen Kriterium und/oder zeitgesteuert.

Zweckmäßig können die Heißreinigungsdauer und die Heißreinigungstemperatur für den Normal-Betriebsmodus und den Eco-Betriebsmodus in Abhängigkeit von einem, insbesondere digitalen, Therapieplan, insbesondere automatisch, angepasst werden.

Zweckmäßig kann die Anpassung der Heißreinigungstemperatur auf die Eco-Heißreinigungstemperatur bzw. die Normal-Heißreinigungstemperatur so erfolgen, dass das Erreichen eines vorbestimmten A₀-Werts, insbesondere trotz der Anpassung dennoch, sichergestellt wird. Da die Reduzierung der Heißreinigungstemperatur unter der Voraussetzung der Erreichung desselben A₀-Werts wie vor der Reduzierung mit einer Verlängerung der Heißreinigungsdauer einhergeht, bietet es sich an, die Reduzierung der Heißreinigungstemperatur dann vorzunehmen, wenn bis zu einer nächsten Therapie ausreichend Zeit vorhanden ist. Dies könnte beispielsweise der Fall sein, wenn mit der nächsten Therapie erst am nächsten Tag zu rechnen ist.

Zweckmäßig ist es denkbar, dass ein Hinweis, insbesondere mittels der Anzeigeeinrichtung der Dialysemaschine, ausgegeben wird, wobei der Hinweis wiedergibt, welcher A₀-Wert gerade erzielt wird.

In weiterer Ausgestaltung der Erfindung basiert der Eco-Betriebsmodus relativ zu dem Normal-Betriebsmodus auf wenigstens einer Eco-Maßnahme. Eine Eco-Maßnahme kann eine Reduzierung der Dialysatflussrate des durch die Dialysemaschine geführten Dialysats sein. Alternativ oder zusätzlich kann eine, insbesondere andere, Eco-Maßnahme eine Reduzierung der Anzeigehelligkeit der Anzeigeeinrichtung der Dialysemaschine sein. Alternativ oder zusätzlich kann eine, insbesondere andere, Eco-Maßnahme eine Anpassung des Farbschemas der Anzeigeeinrichtung sein. Alternativ oder zusätzlich kann eine, insbesondere andere, Eco-Maßnahme eine Deaktivierung der Anzeigeeinrichtung sein. Alternativ oder zusätzlich kann eine, insbesondere andere, Eco-Maßnahme eine Reduzierung der Freispültemperatur bei dem Freispülen der Dialysemaschine sein. Alternativ oder zusätzlich kann eine, insbesondere andere, Eco-Maßnahme eine Verlängerung der Heißreinigungsdauer sowie eine korrespondierende Reduzierung der Heißreinigungstemperatur bei dem Heißreinigen der Dialysemaschine sein. Zweckmäßig erfolgt ein Aktivieren und/oder ein Umschalten wenigstens einer Eco-Maßnahme, insbesondere eines die wenigstens eine Eco-Maßnahme umfassenden Modus, in Abhängigkeit von dem wenigstens einen Kriterium und/oder zeitgesteuert.

In weiterer Ausgestaltung der Erfindung weist das Verfahren einen Schritt auf, gemäß welchem ein Konfigurieren des Eco-Betriebsmodus durch, insbesondere manuelles sowie - alternativ oder zusätzlich - automatisches, Auswählen sowie - alternativ oder zusätzlich - Kombinieren von Eco-Maßnahmen für den Eco-Betriebsmodus, insbesondere in Abhängigkeit von mehreren Kriterien, erfolgt.

In weiterer Ausgestaltung der Erfindung weist die Dialysemaschine eine, insbesondere elektronische, Schnittstelle zur Anbindung an ein übergeordnetes und mit externen Daten gespeistes Steuersystem auf. Dabei weist das Verfahren einen Schritt auf, gemäß welchem ein Eingeben des wenigstens einen Kriteriums sowie - alternativ oder zusätzlich - einer Größe für das wenigstens eine Kriterium über die Schnittstelle erfolgt. Externe Daten können beispielsweise aktuelle Strompreise sein. Dabei kann das übergeordnete Steuersystem zur Verarbeitung der externen Daten eingerichtet sein, um zu erkennen, ob das wenigstens eine Kriterium erfüllt ist.

Alternativ oder zusätzlich kann eine, insbesondere interne, Steuereinrichtung der Dialysemaschine zur Verarbeitung der Daten eingerichtet sein, um zu erkennen, ob das wenigstens eine Kriterium erfüllt ist. Es ist denkbar, dass die Daten mittels des Steuersystems aufbereitet und in aufbereiteter Form der Steuereinrichtung zugeführt werden, um dann von der Steuereinrichtung weiterverarbeitet zu werden. Die Steuereinrichtung kann eine elektronische Mikroprozessoreinrichtung aufweisen.

Eine erfindungsgemäße Dialysemaschine weist eine, insbesondere die, elektronische Steuereinrichtung auf, wobei die Steuereinrichtung zur Durchführung eines erfindungsgemäßen Verfahrens wie voranstehend beschrieben angepasst sowie - alternativ oder zusätzlich - programmiert ist. Die vorstehend erläuterten Vorteile des erfindungsgemäßen Verfahrens übertragen sich somit auch auf die erfindungsgemäße Dialysemaschine. Es versteht sich, dass die Dialysemaschine weitere Mittel zur Durchführung des Verfahrens aufweisen kann.

Ein erfindungsgemäßes Dialysesystem weist eine erfindungsgemäße Dialysemaschine wie vorstehend beschrieben auf. Insofern übertragen sich die vorstehend erwähnten Vorteile der erfindungsgemäßen Dialysemaschine auch auf das erfindungsgemäße Dialysesystem mit einer derartigen Dialysemaschine. Das Dialysesystem weist zudem ein, insbesondere relativ zu der Dialysemaschine, übergeordnetes Steuersystem auf, welches an eine, insbesondere elektronische, Schnittstelle der Dialysemaschine angebunden ist.

Weitere Vorteile und Merkmale der Erfindung ergeben sich aus den Ansprüchen sowie aus der nachfolgenden Beschreibung eines bevorzugten Ausführungsbeispiels der Erfindung, welches anhand der Zeichnungen dargestellt ist. Dabei beziehen sich gleiche Bezugszeichen auf gleiche oder ähnliche oder funktional gleiche Bauteile.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.
- Fig. 1: zeigt in einem Aufbauschema eine Ausführungsform eines erfindungsgemäßen Dialysesystems mit einer Ausführungsform einer erfindungsgemäßen Dialysemaschine bei der Durchführung eines erfindungsgemäß ausgeführten Verfahrens,

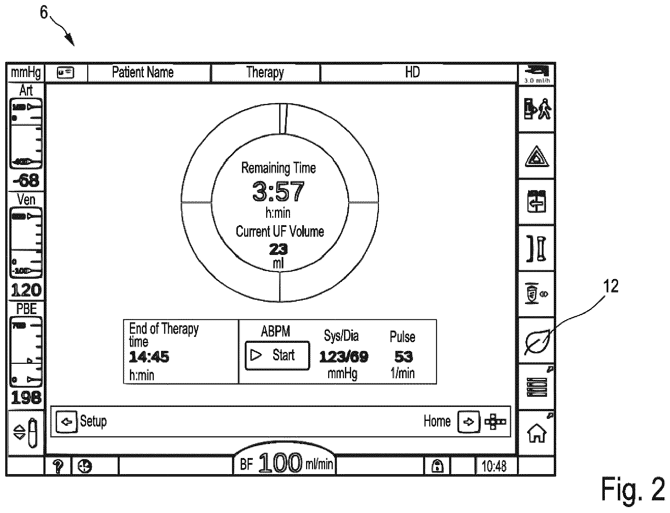

- Fig. 2: schematisch eine Anzeigeoberfläche einer Anzeigeeinrichtung der Dialysemaschine nach Fig. 1,
- Fig. 3: schematisch einen digitalen Therapieplan für einen Betrieb der Dialysemaschine nach Fig. 1, und
- Fig. 4: den Verlauf verschiedener A₀-Werte in einem Zeit-Temperatur-Diagramm.

Eine Dialysemaschine 1 weist einen Normal-Betriebsmodus NM auf. Wenn die Dialysemaschine 1 in ihrem Normal-Betriebsmodus NM betrieben wird, benötigt die Dialysemaschine 1 einen Normal-Bedarf NB an wenigstens einer der Dialysemaschine 1 zugeführten Betriebsressource R.

Die Dialysemaschine 1 weist zudem einen Eco-Betriebsmodus EM auf. Wenn die Dialysemaschine 1 in ihrem Eco-Betriebsmodus EM betrieben wird, benötigt die Dialysemaschine 1 einen Eco-Bedarf EB an der wenigstens einen der Dialysemaschine 1 zugeführten Betriebsressource R. Der Eco-Bedarf EB ist relativ zu dem Normal-Bedarf NB reduziert. Ein etwaiger Bedarf EB, NB an der wenigstens einen Betriebsressource R kann einer für den Betrieb der Dialysemaschine 1 benötigten Menge der Betriebsressource R entsprechen.

Bei der wenigstens einen Betriebsressource R handelt es sich beispielsweise um elektrische Energie E. Alternativ oder zusätzlich handelt es sich bei der wenigstens einen Betriebsressource R um eine Betriebsflüssigkeit L. Die Betriebsflüssigkeit L kann Wasser W aufweisen oder aus Wasser W bestehen.

Die Dialysemaschine 1 weist eine elektronische Steuereinrichtung 2 auf. Die elektronische Steuereinrichtung 2 ist zur Durchführung eines Verfahrens zum Betreiben der Dialysemaschine 1 angepasst und/oder programmiert. Die Dialysemaschine 1 wird gemäß dem Verfahren in Abhängigkeit von wenigstens einem Kriterium K entweder in dem Normal-Betriebsmodus NM oder in dem Eco-Betriebsmodus EM betrieben. Es können mehrere Kriterien K überwacht werden. Falls mehrere Kriterien K überwacht werden, können diese unterschiedlich stark oder gleich gewichtet sein.

Beispielsweise wird das wenigstens eine Kriterium K für ein Aktivieren entweder des Normal-Betriebsmodus NM oder des Eco-Betriebsmodus EM überwacht. Dabei wird entweder der Normal-Betriebsmodus NM oder der Eco-Betriebsmodus EM in Abhängigkeit von dem wenigstens einen Kriterium K aktiviert. Im Anschluss an das Aktivieren entweder des Normal-Betriebsmodus NM oder des Eco-Betriebsmodus EM wird die Dialysemaschine 1 in Abhängigkeit von dem wenigstens einem Kriterium K entweder in dem Normal-Betriebsmodus NM oder in dem Eco-Betriebsmodus EM betrieben. Das Aktivieren entweder des Normal-Betriebsmodus NM oder des Eco-Betriebsmodus EM kann manuell und/oder automatisch erfolgen.

Beispielsweise wird das wenigstens eine Kriterium K für ein Umschalten zwischen dem Normal-Betriebsmodus NM und dem Eco-Betriebsmodus EM überwacht. Dabei wird zwischen dem Normal-Betriebsmodus NM und dem Eco-Betriebsmodus EM in Abhängigkeit von dem wenigstens einen überwachten Kriterium K derart umgeschaltet, dass die Dialysemaschine 1 anschließend in Abhängigkeit von dem wenigstens einen Kriterium K entweder in dem Normal-Betriebsmodus NM oder in dem Eco-Betriebsmodus EM betrieben wird.

Beispielsweise wird zwischen dem Normal-Betriebsmodus NM und dem Eco-Betriebsmodus EM automatisch umgeschaltet. Alternativ oder zusätzlich kann zwischen dem Normal-Betriebsmodus NM und dem Eco-Betriebsmodus EM manuell umgeschaltet werden.

Beispielsweise wird, während die Dialysemaschine 1 betrieben wird, ein Dialysat D durch die Dialysemaschine 1 geführt. Das durch die Dialysemaschine 1 geführte Dialysat D kann eine Dialysatflussrate aufweisen, wobei die Dialysatflussrate beispielsweise 100 ml/min bis 800 ml/min beträgt. Dabei ist die Dialysatflussrate in dem Eco-Betriebsmodus EM beispielsweise kleiner als in dem Normal-Betriebsmodus NM.

Die Dialysatflussrate kann in dem Eco-Betriebsmodus EM einem 1,0-Fachen bis 1,6-Fachen, insbesondere einem 1,0-Fachen bis 1,2-Fachen, einer Blutflussrate mittels der Dialysemaschine 1 dialysierten Bluts B entsprechen.

Beispielsweise werden das Dialysat D und das dialysierte Blut B, vorliegend im Gegenstrom, durch einen Dialysator 13 der Dialysemaschine 1 geführt.

Die Dialysemaschine 1 weist beispielsweise eine elektronische Anzeigeeinrichtung 6 auf. Die Anzeigeeinrichtung 6 ist vorliegend berührungssensitiv ausgebildet. Die Anzeigeeinrichtung 6 kann ein Touch-Screen aufweisen. Die Anzeigeeinrichtung 6 ist beispielsweise zur Visualisierung eines momentanen Betriebsmodus NM, EM der Dialysemaschine 1 ausgebildet.

In dem Eco-Betriebsmodus EM kann eine Anzeigehelligkeit der Anzeigeeinrichtung 6 geringer sein als in dem Normal-Betriebsmodus NM. Alternativ oder zusätzlich kann ein Farbschema der Anzeigeeinrichtung 6 in dem Eco-Betriebsmodus EM gegenüber dem Normal-Betriebsmodus NM verändert sein. Alternativ oder zusätzlich kann die Anzeigeeinrichtung 6 in dem Eco-Betriebsmodus EM deaktiviert sein, wohingegen die Anzeigeeinrichtung 6 in dem Normal-Betriebsmodus NM aktiviert ist.

Beispielsweise weist die Anzeigeeinrichtung 6 ein OLED-Display auf. Das Farbschema des OLED-Displays kann in dem Eco-Betriebsmodus EM auf "schwarz-weiß" eingestellt sein. Demgegenüber kann das Farbschema des OLED-Displays in dem Normal-Betriebsmodus NM zum farbigen Anzeigen und/oder auf "bunt" eingestellt sein. Bei dem OLED-Display kann die Farbe Schwarz für das schwarz-weiße Farbschema des Eco-Betriebsmodus EM durch unbeleuchtete und/oder ausgeschaltete Pixel des OLED-Displays dargestellt werden.

Beispielsweise weist die Dialysemaschine 1 eine Sensoreinrichtung 3 auf. Die Sensoreinrichtung 3 kann - wie vorliegend - zum Detektieren einer Größe G für das wenigstens eine Kriterium K ausgebildet sein.

Vorliegend weist die Sensoreinrichtung 3 einen Helligkeitssensor 7 zum Detektieren einer Umgebungshelligkeit der Dialysemaschine 1 auf. Dabei kann die Umgebungshelligkeit eine Größe G für das wenigstens eine Kriterium K sein. Alternativ oder zusätzlich kann das wenigstens eine Kriterium K ein vorbestimmter Schwellwert der Umgebungshelligkeit sein.

Die Sensoreinrichtung 3 umfasst vorliegend einen Näherungssensor 8 zum Detektieren einer sich der Dialysemaschine 1 nähernden Person. Dabei kann ein Abstand der Person zu der Dialysemaschine 1 eine Größe G für das wenigstens eine Kriterium K sein. Alternativ oder zusätzlich kann das wenigstens eine Kriterium K ein vorbestimmter Schwellwert des Abstands sein.

Die Sensoreinrichtung 3 weist vorliegend einen Berührungssensor 12 zum Detektieren einer berührenden, insbesondere manuellen, Eingabe der Größe G für das wenigstens eine Kriterium K auf. Der Berührungssensor 12 kann von der berührungssensitiven Anzeigeeinrichtung 6 umfasst sein. Vorliegend ist der Berührungssensor 12 integral und funktional von der berührungssensitiven Anzeigeeinrichtung 6 umfasst. Beispielsweise kann der Berührungssensor 12 durch einen Schaltflächenabschnitt, insbesondere eines Touch-Screens, der berührungssensitiven Anzeigeeinrichtung 6 ausgebildet sein.

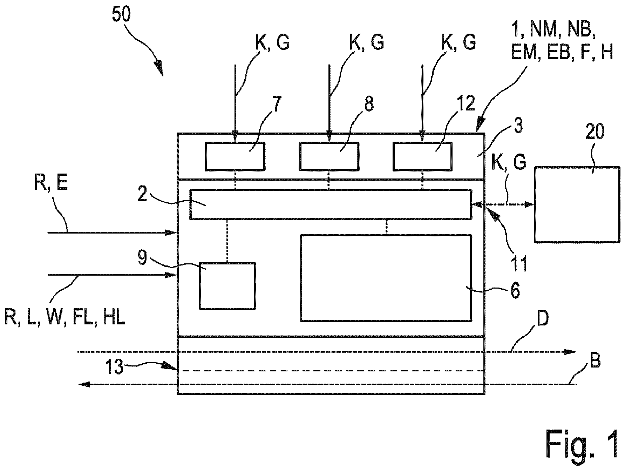

Es versteht sich, dass bei von jener der Fig. 1 abweichenden Ausführungsformen auf einzelne oder mehrere der Sensoren der Sensoreinrichtung 3 verzichtet sein kann.

Die Dialysemaschine 1 weist beispielsweise einen Freispülmodus F zum Freispülen der Dialysemaschine 1 mit Freispülflüssigkeit FL auf. Die Freispülflüssigkeit FL kann Betriebsflüssigkeit L aufweisen oder sein. Der Freispülmodus F ist in dem Normal-Betriebsmodus NM und in dem Eco-Betriebsmodus EM, insbesondere je wahlweise, aktivierbar.

Wenn der Freispülmodus F während des Normal-Betriebsmodus NM aktiviert wird, wird die Freispülflüssigkeit FL mit einer Normal-Freispültemperatur und mit einer Freispülflüssigkeitsflussrate durch die Dialysemaschine 1 geführt. Vorliegend weist die Dialysemaschine 1 eine elektrische Heizeinrichtung 9 auf, mittels welcher der Freispülflüssigkeit FL bei Aktivierung des Freispülmodus F während des Normal-Betriebsmodus NM Wärme zugeführt wird. Die Normal-Freispültemperatur beträgt beispielsweise 40 °C. Die Freispülflüssigkeitsflussrate beträgt beispielsweise 1l/min.

Wenn der Freispülmodus F während des Eco-Betriebsmodus EM aktiviert wird, wird die Freispülflüssigkeit mit einer gegenüber der Normal-Freispültemperatur reduzierten Eco-Freispültemperatur und mit der - vorliegend selben - Freispülflüssigkeitsflussrate durch die Dialysemaschine 1 geführt. Vorliegend wird der Freispülflüssigkeit FL bei Aktivierung des Freispülmodus F im Eco-Betriebsmodus EM keine Wärme mittels der elektrischen Heizeinrichtung 9 zugeführt. Die Eco-Freispültemperatur kann beispielsweise 15 °C betragen. Insbesondere entspricht die Eco-Freispültemperatur einer Freispülflüssigkeits-Einlauftemperatur stromauf der Dialysemaschine 1.

Beispielsweise weist die Dialysemaschine 1 einen Heißreinigungsmodus H zum Heißreinigen der Dialysemaschine 1 mit einer Heißreinigungsflüssigkeit HL auf. Die Heißreinigungsflüssigkeit HL kann Betriebsflüssigkeit L aufweisen oder Betriebsflüssigkeit L sein. Der Heißreinigungsmodus H ist in dem Normal-Betriebsmodus NM und in dem Eco-Betriebsmodus EM, beispielsweise je wahlweise, aktivierbar.

Wenn der Heißreinigungsmodus H während des Normal-Betriebsmodus NM aktiviert wird, wird die Heißreinigungsflüssigkeit HL mit einer Normal-Heißreinigungstemperatur und mit einer Normal-Heißreinigungsdauer durch die Dialysemaschine 1 geführt. Dabei kann der Heißreinigungsflüssigkeit HL mittels der Heizeinrichtung 9 der Dialysemaschine 1 Wärme zugeführt werden, um die Heißreinigungsflüssigkeit HL auf die Normal-Heißreinigungstemperatur zu erhitzen.

Wenn der Heißreinigungsmodus H während des Eco-Betriebsmodus EM aktiviert wird, wird die Heißreinigungsflüssigkeit HL mit einer gegenüber der Normal-Heißreinigungstemperatur reduzierten Eco-Heißreinigungstemperatur und mit einer gegenüber der Normal-Heißreinigungsdauer verlängerten Eco-Heißreinigungsdauer durch die Dialysemaschine 1 geführt. Insbesondere wird bei Aktivierung des Heißreinigungsmodus H im Eco-Betriebsmodus EM der Heißreinigungsflüssigkeit HL Wärme mittels der Heizeinrichtung 9 zugeführt, insbesondere jedoch weniger Wärme als bei Aktivierung des Heißreinigungsmodus H im Normal-Betriebsmodus NM.

Beispielsweise können die Heißreinigungstemperatur und die Heißreinigungsdauer für den Normal-Betriebsmodus NM und für den Eco-Betriebsmodus EM jeweils so ausgewählt sein, dass sowohl in dem Normal-Betriebsmodus NM als auch in dem Eco-Betriebsmodus EM ein selber A₀-Wert erreicht werden kann. Die Heißreinigungsdauer zur Erzielung eines vorbestimmten A₀-Werts nimmt insbesondere mit sinkender Heißreinigungsdauer exponentiell zu.

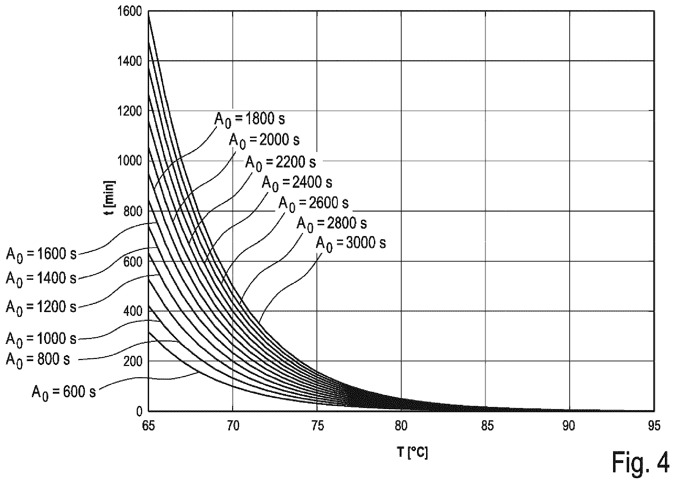

Kombinationen für Heißreinigungstemperaturen und Heißreinigungsdauern zur Erreichung eines jeweiligen A₀-Werts können beispielhaft dem in Fig. 4 gezeigten Diagramm entnommen werden. Beispielsweise kann demgemäß ein A₀-Wert von 600 s durch eine Heißreinigungstemperatur von 90 °C bei einer Heißreinigungsdauer von 1 min oder durch eine Heißreinigungstemperatur von 80 °C bei einer Heißreinigungsdauer von 10 min oder durch eine Heißreinigungstemperatur von 70 °C bei einer Heißreinigungsdauer von 100 min erreicht werden. Falls der A₀-Wert zu 3.000 s festgelegt wird, kann die Heißreinigungstemperatur beispielsweise 90 °C bei einer Heißreinigungsdauer von 5 min oder 80 °C bei einer Heißreinigungsdauer von 50 min oder 70 °C bei einer Heißreinigungsdauer von 500 min betragen.

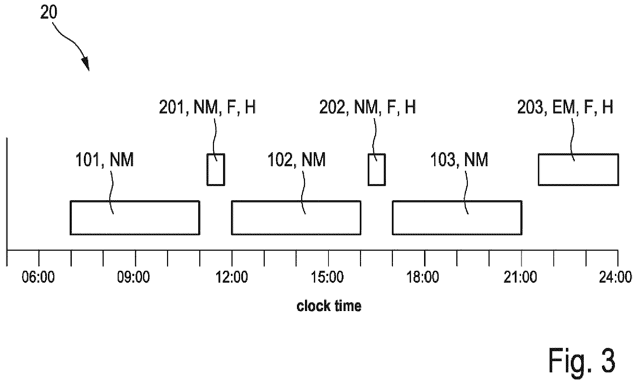

Beispielsweise kann die Anpassung der Heißreinigungsdauer und der Heißreinigungstemperatur in Abhängigkeit von einem Therapieplan erfolgen. Der Therapieplan kann digital vorliegen, wie beispielsweise anhand der Fig. 3 veranschaulicht. Dabei sind drei Therapien 101, 102, 103 erkennbar, die hintereinander an demselben Tag durchgeführt werden. Zwischen der Therapie 101 und der Therapie 102 sowie zwischen der Therapie 102 und der Therapie 103 sind lediglich Zeitintervalle 201, 202 für die Normal-Heißreinigungsdauer vorhanden, so dass dort der Heißreinigungsmodus H während des Normal-Betriebsmodus NM aktiviert wird. Im direkten Anschluss an die Therapie 103 erfolgt dagegen vorliegend keine weitere Therapie am selben Tag, so dass über Nacht ein Zeitintervall 203 für die Eco-Heißreinigungsdauer zur Verfügung steht, weshalb vorliegend im Anschluss an die Therapie 103 der Heißreinigungsmodus H während des Eco-Betriebsmodus EM aktiviert wird.

Der Eco-Betriebsmodus EM basiert beispielsweise gegenüber dem Normal-Betriebsmodus NM auf wenigstens einer Eco-Maßnahme. Dabei kann eine Eco-Maßnahme die Reduzierung der Dialysatflussrate des durch die Dialysemaschine 1 geführten Dialysats D sein. Eine andere Eco-Maßnahme kann die Reduzierung der Anzeigehelligkeit der Anzeigeeinrichtung 6 der Dialysemaschine 1 sein. Eine andere Eco-Maßnahme kann die Anpassung des Farbschemas der Anzeigeeinrichtung 6 sein. Eine andere Eco-Maßnahme kann die Deaktivierung der Anzeigeeinrichtung 6 sein. Eine andere Eco-Maßnahme kann die Reduzierung der Freispültemperatur bei dem Freispülen der Dialysemaschine 1 sein. Eine andere Eco-Maßnahme kann die Verlängerung der Heißreinigungsdauer bei korrespondierender Reduzierung der Heißreinigungstemperatur bei dem Heißreinigen der Dialysemaschine 1 sein.

Beispielsweise kann der Eco-Betriebsmodus EM durch, beispielsweise manuelles und/der automatisches, Auswählen und/oder Kombinieren von Eco-Maßnahmen für den Eco-Betriebsmodus EM konfiguriert werden. Insbesondere kann der Eco-Betriebsmodus EM in Abhängigkeit von mehreren Kriterien K konfiguriert werden.

Die Dialysemaschine 1 weist beispielsweise eine, vorliegend elektronische, Schnittstelle 11 auf, wobei die Schnittstelle 11 zur Anbindung an ein übergeordnetes und mit externen Daten gespeistes Steuersystem 20 ausgebildet ist. Dabei kann das wenigstens eine Kriterium K und/oder eine Größe G für das wenigstens eine Kriterium K über die Schnittstelle 11 eingegeben werden.

Externe Daten können beispielsweise aktuelle Strompreise sein, d. h. Preise für die wenigstens eine Betriebsressource R in Form elektrischer Energie. Dabei kann das übergeordnete Steuersystem 20 zur Verarbeitung der externen Daten eingerichtet sein, um zu erkennen, ob das wenigstens eine Kriterium K erfüllt ist. Alternativ oder zusätzlich kann die, insbesondere interne, Steuereinrichtung 2 der Dialysemaschine 1 zur Verarbeitung der Daten eingerichtet sein, um zu erkennen, ob das wenigstens eine Kriterium K erfüllt ist. Es ist denkbar, dass die Daten mittels des Steuersystems 20 aufbereitet und in aufbereiteter Form der Steuereinrichtung 2 zugeführt werden, um dann von der Steuereinrichtung 2 weiterverarbeitet zu werden.

Die Dialysemaschine 1 ist vorliegend von einem Dialysesystem 50 aufgewiesen. Dabei weist das Dialysesystem 50 zudem das der Dialysemaschine 1 übergeordnete Steuersystem 20 auf, wobei das Steuersystem 20 an die Schnittstelle 11 der Dialysemaschine 1 angebunden ist.

Die externen Daten können dem übergeordneten Steuersystem 20 beispielsweise kontinuierlich zugeführt werden. Das Steuersystem 20 kann also mit den externen Daten kontinuierlich gespeist sein. Das Steuersystem 20 kann auf Grundlage der externen Daten, insbesondere automatisch, entscheiden, ob und/oder welche Eco-Maßnahme eingeleitet werden soll. Dabei kann beispielsweise beim Überschreiten eines vorbestimmten Strompreis-Schwellwerts eine jeweilige Eco-Maßnahme automatisch aktiviert werden. Alternativ oder zusätzlich können Modelle zur prädiktiven Analyse verwendet werden, die zum Beispiel basierend auf zurückliegenden Entwicklungen des Strompreises eine zukünftige Preisentwicklung vorhersagen können.

## Patentansprüche

1. Verfahren zum Betreiben einer Dialysemaschine (1), wobei die Dialysemaschine (1) aufweist:
- einen Normal-Betriebsmodus (NM), in welchem die Dialysemaschine (1) einen Normal-Bedarf (NB) an wenigstens einer der Dialysemaschine (1) zugeführten Betriebsressource (R) benötigt, und
- einen Eco-Betriebsmodus (EM), in welchem die Dialysemaschine (1) einen relativ zu dem Normal-Bedarf (NB) reduzierten Eco-Bedarf (EB) an der wenigsten einen der Dialysemaschine (1) zugeführten Betriebsressource (R) benötigt;
wobei das Verfahren den Schritt aufweist:
- Betreiben der Dialysemaschine (1) in Abhängigkeit von wenigstens einem Kriterium (K) entweder in dem Normal-Betriebsmodus (NM) oder in dem Eco-Betriebsmodus (EM).

2. Verfahren nach Anspruch 1, **dadurch gekennzeichnet, dass**
das Verfahren die Schritte aufweist:
- Überwachen des wenigstens einen Kriteriums (K) für ein Aktivieren entweder des Normal-Betriebsmodus (NM) oder des Eco-Betriebsmodus (EM), und
- Aktivieren entweder des Normal-Betriebsmodus (NM) oder des Eco-Betriebsmodus (EM) in Abhängigkeit von dem wenigstens einen Kriterium (K), um die Dialysemaschine (1) anschließend in Abhängigkeit von dem wenigstens einen Kriterium (K) entweder in dem Normal-Betriebsmodus (NM) oder in dem Eco-Betriebsmodus (EM) zu betreiben.

3. Verfahren nach Anspruch 1 oder 2, **dadurch gekennzeichnet, dass**
das Verfahren die Schritte aufweist:
- Überwachen des wenigstens einen Kriteriums (K) für ein Umschalten zwischen dem Normal-Betriebsmodus (NM) und dem Eco-Betriebsmodus (EM), und
- Umschalten zwischen dem Normal-Betriebsmodus (NM) und dem Eco-Betriebsmodus (EM) in Abhängigkeit von dem wenigstens einen überwachten Kriterium (K), so dass die Dialysemaschine (1) anschließend in Abhängigkeit von dem wenigstens einen Kriterium (K) entweder in dem Normal-Betriebsmodus (NM) oder in dem Eco-Betriebsmodus (EM) betrieben wird.

4. Verfahren nach einem der Ansprüche 1 bis 3, **dadurch gekennzeichnet, dass**
- ein Umschalten zwischen dem Normal-Betriebsmodus (NM) und dem Eco-Betriebsmodus (EM) automatisch erfolgt, und/oder dass
- das Umschalten zwischen dem Normal-Betriebsmodus (NM) und dem Eco-Betriebsmodus (EM) manuell erfolgt.

5. Verfahren nach einem der vorhergehenden Ansprüche, **dadurch gekennzeichnet, dass**
- bei dem Betreiben der Dialysemaschine (1) ein Dialysat (D) mit einer Dialysatflussrate, insbesondere von 100 ml/min bis 800 ml/min, durch die Dialysemaschine (1) geführt wird,
- wobei die Dialysatflussrate in dem Eco-Betriebsmodus (EM) kleiner ist als in dem Normal-Betriebsmodus (NM),
- insbesondere wobei in dem Eco-Betriebsmodus (EM) die Dialysatflussrate einem 1,0-Fachen bis 1,6-Fachen einer Blutflussrate mittels der Dialysemaschine (1) dialysierten Bluts (B) entspricht.

6. Verfahren nach einem der vorhergehenden Ansprüche, **dadurch gekennzeichnet, dass**
- die Dialysemaschine (1) eine, insbesondere berührungssensitive, elektronische Anzeigeeinrichtung (6) zur Visualisierung eines momentanen Betriebsmodus (NM, EM) der Dialysemaschine (1) aufweist,
- wobei in dem Eco-Betriebsmodus (EM) relativ zu dem Normal-Betriebsmodus (NM):
- eine Anzeigehelligkeit der Anzeigeeinrichtung (6) geringer ist, und/oder
- ein Farbschema der Anzeigeneinrichtung (6) angepasst ist, und/oder
- die Anzeigeeinrichtung (6) deaktiviert ist.

7. Verfahren nach einem der vorhergehenden Ansprüche, **dadurch gekennzeichnet, dass**
- die Dialysemaschine (1) eine Sensoreinrichtung (3), insbesondere zum Detektieren einer Größe (G) für das wenigstens eine Kriterium (K), aufweist,
- wobei die Sensoreinrichtung (3) aufweist:
- einen Helligkeitssensor (7) zum Detektieren einer Umgebungshelligkeit der Dialysemaschine (1), insbesondere wobei die Umgebungshelligkeit eine Größe (G) für das wenigstens eine Kriterium (K) ist und/oder das wenigstens eine Kriterium (K) ein vorbestimmter Schwellwert der Umgebungshelligkeit ist, und/oder
- einen Näherungssensor (8) zum Detektieren einer sich der Dialysemaschine (1) nähernden Person, insbesondere wobei ein Abstand der Person zu der Dialysemaschine (1) eine Größe (G) für das wenigstens eine Kriterium (K) ist und/oder das wenigstens eine Kriterium (K) ein vorbestimmter Schwellwert des Abstands ist, und/oder
- einen Berührungssensor (12) zum Detektieren einer berührenden, insbesondere manuellen, Eingabe einer Größe (G) für das wenigstens eine Kriterium (K).

8. Verfahren nach einem der vorhergehenden Ansprüche, **dadurch gekennzeichnet, dass**
- die Dialysemaschine (1) einen in dem Normal-Betriebsmodus (NM) und in dem Eco-Betriebsmodus (EM), insbesondere je wahlweise, aktivierbaren Freispülmodus (F) zum Freispülen der Dialysemaschine (1) mit Freispülflüssigkeit (FL), insbesondere mit oder aus Betriebsflüssigkeit (L), aufweist,
- wobei - wenn der Freispülmodus (F) bei dem Betreiben der Dialysemaschine (1) in dem Normal-Betriebsmodus (NM) aktiviert wird - die Freispülflüssigkeit (FL), insbesondere bei Wärmezufuhr mittels einer elektrischen Heizeinrichtung (9) der Dialysemaschine (1), mit einer Normal-Freispültemperatur und mit einer Freispülflüssigkeitsflussrate durch die Dialysemaschine (1) geführt wird,
- wobei - wenn der Freispülmodus (F) bei dem Betreiben der Dialysemaschine (1) in dem Eco-Betriebsmodus (EM) aktiviert wird - die Freispülflüssigkeit (FL), insbesondere ohne Wärmezufuhr mittels der elektrischen Heizeinrichtung (9), mit einer gegenüber der Normal-Freispültemperatur reduzierten Eco-Freispültemperatur und mit der Freispülflüssigkeitsflussrate durch die Dialysemaschine (1) geführt wird.

9. Verfahren nach einem der vorhergehenden Ansprüche, **dadurch gekennzeichnet, dass**
- die Dialysemaschine (1) einen in dem Normal-Betriebsmodus (NM) und in dem Eco-Betriebsmodus (EM), insbesondere je wahlweise, aktivierbaren Heißreinigungsmodus (H) zum Heißreinigen der Dialysemaschine (1) mit einer Heißreinigungsflüssigkeit (HL), insbesondere mit oder aus Betriebsflüssigkeit (L), aufweist,
- wobei - wenn der Heißreinigungsmodus (H) bei dem Betreiben der Dialysemaschine (1) in dem Normal-Betriebsmodus (NM) aktiviert wird - die Heißreinigungsflüssigkeit (HL), insbesondere bei Wärmezufuhr mittels einer elektrischen Heizeinrichtung (9) der Dialysemaschine (1), mit einer Normal-Heißreinigungstemperatur und mit einer Normal-Heißreinigungsdauer durch die Dialysemaschine (1) geführt wird,
- wobei - wenn der Heißreinigungsmodus (H) bei dem Betreiben der Dialysemaschine (1) in dem Eco-Betriebsmodus (EM) aktiviert wird - die Heißreinigungsflüssigkeit (HL), insbesondere bei Wärmezufuhr mittels der elektrischen Heizeinrichtung (9), mit einer gegenüber der Normal-Heißreinigungstemperatur reduzierten Eco-Heißreinigungstemperatur und mit einer gegenüber der Normal-Heißreinigungsdauer verlängerten Eco-Heißreinigungsdauer durch die Dialysemaschine (1) geführt wird.

10. Verfahren nach einem der vorhergehenden Ansprüche, **dadurch gekennzeichnet, dass**
- der Eco-Betriebsmodus (EM) relativ zu dem Normal-Betriebsmodus (NM) auf wenigstens eine der folgenden Eco-Maßnahmen basiert:
- Reduzierung einer Dialysatflussrate eines durch die Dialysemaschine (1) geführten Dialysats (D),
- Reduzierung einer Anzeigehelligkeit einer Anzeigeeinrichtung (6) der Dialysemaschine (1),
- Anpassung eines Farbeschemas der Anzeigeeinrichtung (6),
- Deaktivierung der Anzeigeeinrichtung (6),
- Reduzierung einer Freispültemperatur bei einem Freispülen der Dialysemaschine (1),
- Verlängerung einer Heißreinigungsdauer sowie korrespondierende Reduzierung einer Heißreinigungstemperatur bei einem Heißreinigen der Dialysemaschine (1).

11. Verfahren nach einem der vorhergehenden Ansprüche, **dadurch gekennzeichnet, dass** das Verfahren den Schritt aufweist:
- Konfigurieren des Eco-Betriebsmodus (EM) durch, insbesondere manuelles und/oder automatisches, Auswählen und/oder Kombinieren von Eco-Maßnahmen für den Eco-Betriebsmodus (EM), insbesondere in Abhängigkeit von mehreren Kriterien (K).

12. Verfahren nach einem der vorhergehenden Ansprüche, **dadurch gekennzeichnet, dass**
- die Dialysemaschine (1) eine, insbesondere elektronische, Schnittstelle (11) zur Anbindung an ein übergeordnetes und mit externen Daten gespeistes Steuersystem (20) aufweist;
wobei das Verfahren den Schritt aufweist:
- Eingeben des wenigstens einen Kriteriums (K) und/oder einer Größe (G) für das wenigstens eine Kriterium (K) über die Schnittstelle (11).

13. Dialysemaschine (1), aufweisend
- eine elektronische Steuereinrichtung (2), welche zur Durchführung eines Verfahrens nach einem der vorhergehenden Ansprüche angepasst und/oder programmiert ist.

14. Dialysesystem (50), aufweisend:
- eine Dialysemaschine (1) nach Anspruch 13, und
- ein übergeordnetes Steuersystem (20), welches an eine, insbesondere elektronische, Schnittstelle (11) der Dialysemaschine (1) angebunden ist.
